# EUROPEAN PATENT APPLICATION

(11) **EP 2 902 497 A1**
(43) Date of publication of application: **05.08.2015**
(21) Application number: 13840641.8
(22) Date of filing: 21.06.2013
(51) Int. Cl.: C12Q 1/04, C12Q 1/30

(54) **USE OF A HYDROGEN PEROXIDE COMPOSITION FOR DETECTING BIOFILMS**

(30) Priority: 25.09.2012 ES 201231482
(71) Applicant: Itram Higiene, S.L., 08500 Vic (Barcelona) (ES)
(72) Inventor: SALAS VÁZQUEZ, Dora Isela, 08029 Barcelona (ES); RODRÍGUEZ JEREZ, Juan José, 08195 Sant Cugat del Vallès (ES); OSSET HERNÁNDEZ, Miguel, 08445 Cànoves I Samalús (ES); MONTAÑEZ IZQUIERDO, Vanessa, 08032 Barcelona (ES); FERNÁNDEZ BLANCO, Minerva, 08016 Barcelona (ES); FORNS AGUDO, Isabel, 08100 Mollet del Vallès (ES)
(74) Representative: Gallego Jiménez, José Fernando
(86) International application number: PCT/ES2013/070401
(87) International publication number: WO 2014/049182

(57) **Abstract**

The invention concerns the use of a hydrogen peroxide composition for detecting biofilms. The composition also comprises a thickener and is particularly suitable for rapidly and efficiently detecting biofilms *in situ* on surfaces exposed to microbial contamination, for example in the agroalimentary industry and the pharmaceutical and hospital fields. The invention also concerns a method for detecting biofilms using this composition.

## Description

### Technical Field

The present invention belongs to the field of surface hygiene and refers to a method for the detection of biofilms by employing a composition comprising hydrogen peroxide.

### State of the Art

A biofilm is an aggregate of microorganisms composed of a population of cells growing adhered to each other, anchored to a surface, and embedded in a matrix of extracellular polymeric substances (EPS) produced by the biofilm itself. The EPS can contain polysaccharides, proteins, phospholipids, teichoic and nucleic acids, and other polymeric substances hydrated with up to 97% of water.

The biofilm can be composed either of microorganism monocultures, or of cultures of different species, as well as of a mixture of phenotypes of a given species. The formation of biofilms is not restricted to a particular group of microorganisms but nowadays it is considered that, in the appropriate environmental conditions, all microorganisms are capable of forming biofilms on any kind of surface.

The advantages provided by a biofilm to the life of microorganisms are numerous, mainly the availability of more stable environmental conditions, a reserve of nutrients, an anchoring point, and greater resistance to desiccation. Likewise, the biofilm also provides a greater resistance to microorganisms against the action of disinfectants.

Therefore, in practice, a biofilm is a barrier between microorganisms and disinfectants, antibiotics or biocides, as described in the article Bredholt et al., Microbial methods for assessment of cleaning and disinfection of food-processing surfaces cleaned in a low-pressure system, Eur. Food Res. Technol., 1999, 209 (2), 145-152.

Although most species of bacteria have the ability to form biofilms, some genus form them more easily and quickly than others, such as *Pseudomonas, Listeria, Enterobacter, Flavobacterium, Alcaligenes, Staphylococcus,* and *Bacillus.* Biofilm formation is not exclusive of bacteria, as yeasts can also produce them, for example, *Saccharomyces cerevisiae,* present in the wine industry.

The presence of biofilms is particularly undesirable in those areas where hygiene is a fundamental aspect, as for example, in the food and beverages manufacturing processes, or in the pharmaceutical and hospital environments.

However, the presence of biofilms has been determined in such diverse areas as, for example, in the medical environment, involved in the pathogenesis of various infectious diseases and in the formation of the dental plaque; in hospitals, as contaminants on different surfaces, particularly in medical devices, as well as in catheters, implants or prostheses; in the food industry the proliferation of biofilms on surfaces, pipes and equipment, on any kind of material, poses a risk of contamination of the finished product, and can represent an important public health problem; in various water systems, such as in industrial refrigeration systems and drinking water pipes; in the domestic environment, mainly in bathrooms and kitchens; among other possible examples.

In the biofilms found in the hospital environment, usually the Gram-positive bacteria predominate, mainly of the Staphylococcus genus, although the infections due to Gram-negative bacteria and fungi are usually more serious. The most common fungal infections are often caused by pathogenic species of *Candida,* particularly *C. albicans, C. dubliniensis, C. tropicalis* and *C. parapsilosis,* as described in the articles Hawser et al., Biofilm formation by Candida species on the surface catheter of materials in vitro, Infection Immunity, 1994, 62 (3), 915-921, and Jabra-Rizk et al., Fungal biofilms and drug resistance, Emerging Infect. Dis., 2004, 10 (1), 14-19.

The most relevant species involved in biofilm formation in the food production environment and that particularly endanger the food security are mainly *Listeria monocytogenes, Salmonella spp., Escherichia coli, Cronobacter spp., Pseudomonas spp., Campylobacter jejuni* and *Bacillus spp,* as described in the articles Mattila-Sandholm et al., Biofilm in the industry: A review, Food Rev. Int., 1992, 8 (4), 573-603, Lee Wong et al., Biofilm in Food Processing Environments, J. Dairy Sci., 1998, 81 (10), 2765-2770, and Jo et al., Maturation and survival of Cronobacter biofilms on silicone, polycarbonate, stainless steel and UV light and ethanol after immersion treatments, J. Food Protection, 2010, 73 (5), 952-956.

The surfaces are one of the most frequent ways of food contamination, both in the food industry and in catering services, or also at home. The food contact surfaces include those having direct contact with food, as well as those from which there is a pouring over the food or over food contacting surfaces, usually during the course of operations. Utensils and surfaces of the equipment are also included.

Thus, the food industry requires a strict adherence to the established rules of hygiene and rigorous application of the Hazard Analysis and Critical Control Points (HACCP) system along the food chain, as described in the article Panisello et al., Application of foodborne disease outbreak data in the development and maintenance of HACCP systems, Int. J. Food Microbiol., 2000, 59 (3), 221-234.

In this context, it is essential to carry out an effective control of the formation of biofilms on the food contact surfaces, since when fragments of a biofilm with viable cells are detached, they can be dispersed and act as a matrix for the attachment of new cells, which will give rise to a new biofilm, with the subsequent contamination of the surfaces.

One of the key factors for implementing these hygiene systems is to have a fast and effective method to monitor their effectiveness. It is therefore essential to have methods capable of detecting biofilms, which can act as auxiliaries for the establishment of the Critical Control Points (CCP) on the surfaces of the agri-food industry.

In the prior art a number of methods are known for the study and monitoring of biofilms from various environments, based on, for example, techniques such as fluorescence microscopy, scanning electron microscopy (SEM), differential interference contrast microscopy (DIC), transmission electron microscopy (TEM) or, more recently, the confocal laser scanning microscopy (CLSM) in conjunction with image analysis have contributed to a better visual understanding of the biofilm architecture, both static and dynamic, as described in the article Schlapp et al., Development of 3D architecture of uropathogenic Proteus mirabilis batch culture, J. Microbiol. Methods, 2011, 87, 234-240.

However, one of the most widely used methods in the study and detection of biofilm is the tissue culture plate method (TCP), which is based on performing cultures in plates and subsequently analyzing the formed biofilm by techniques such as ELISA, after fixation and staining of the biofilm, as described in the article Christensen et al., Adherence of coagulase-negative staphylococci to plastic tissue culture plates: a quantitative model for the adherence of staphylococci to medical devices, J. Clin. Microbiol., 1985, 22 (6), 996-1006.

At present, the methods available for the determination of biofilms on surfaces, for example in the food industry, are the traditional and rapid microbiological control methods. Traditional methods include plate culture or application-imprint methods (Rodac, slides and Petrifilm) that, although reliable and efficient, require at least from several days to one week for the results to be obtained, so they are inappropriate as a quick monitoring tool for the HACCP.

The rapid methods are grouped into three categories: qualitative, quantitative and identification methods. Although all three give a "quick" result, only the qualitative analysis allows giving a result *in situ* and immediately. This quick result in the detection of biofilms is one of the most valuable premises at industrial level, as it allows identifying, containing and recovering quickly from an episode of localized contamination. Some examples of rapid methods are: ELISA (immunological), Detection of ATP by bioluminescence (enzymatic) Direct Epifluorescence Microscopy (DEM) and Real Time PCR (molecular biology).

Likewise, also other methods have been described for this purpose, as the use of certain substances that are capable of selectively staining the extracellular polymer matrix of the biofilm. Thus, for example, the Japanese patent application JP-A-2005210997 relates to a method for detecting biofilm on surfaces, preferably in the food industry field, in order to improve the cleaning and disinfection thereof, and that consists in the staining of the biofilm with a composition comprising *Monascus Red* dye.

There are available in the market some commercial products also based on the use of dyes selective for the biofilm polymeric matrix, for example, the "Biofilm detector kit" marketed by the company Realco and "Biofilms detection Test TBF 300 "marketed by the company Betelgeux.

However, most of the methods described in the prior art are not suitable or completely satisfactory for the immediate detection of biofilms in relatively large surfaces, particularly in the agri-food industry or in the pharmaceutical and hospital environments, and especially during the routine tasks of cleaning and disinfection either for their lack of efficacy in the detection, or because they do not allow having the results quickly, or due to their high cost.

Thus, it is apparent that there remains the need to have a method for detecting biofilms on surfaces of all kinds, especially in the agri-food industry field or in the pharmaceutical and hospital environments, that is effective in detecting biofilms in a quick and precise way, that is easy to use and does not require complex instrumentation, that is applicable to large surface areas and that is economical to encourage its widespread application, thus allowing a better control of the presence of biofilms and consequently also a better monitoring of the effectiveness of the sanitation systems, which in turn would allow applying the corrective measures in the same moment that the determinations are made.

### Object of the Invention

The object of the present invention is the use of a hydrogen peroxide composition for detecting biofilms.

Another aspect of the present invention is a method for detecting biofilms using this composition.

### Description of the figures

Figure 1:

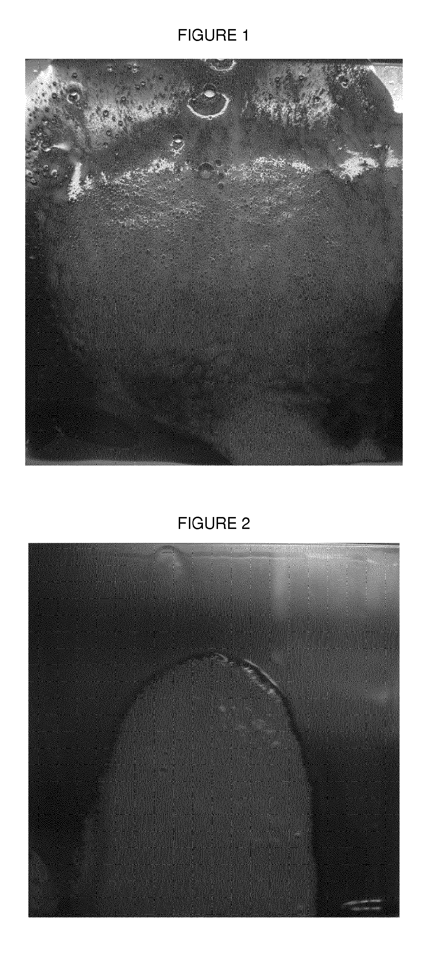

   Figure 1 shows a photograph of the result of the application of the method according to the present invention, for the detection of biofilms of *Pseudomonas aeruginosa* on a stainless steel surface disposed in horizontal position.
Figure 2:
   Figure 2 shows a photograph of the result of the application of the method according to the present invention, for the detection of biofilms of *Pseudomonas aeruginosa* on a stainless steel surface disposed in a vertical position.
Figure 3:

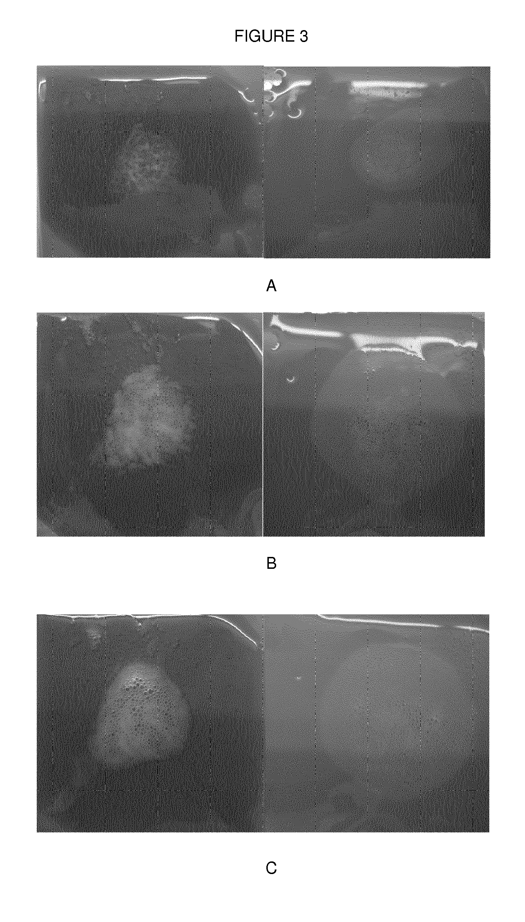

   Figure 3 shows a photograph of the result of the application of the method according to the present invention (left image), or after the application of an aqueous hydrogen peroxide solution (right image), for the detection of biofilms of *Pseudomonas aeruginosa* on surfaces coated with epoxy paint, disposed horizontally, after 30 seconds of reaction (3A).
Figure 3B shows a photograph of the same surfaces after 5 minutes of reaction.
Figure 3C shows a photograph of the same surfaces after 15 minutes of reaction.
Figure 4:

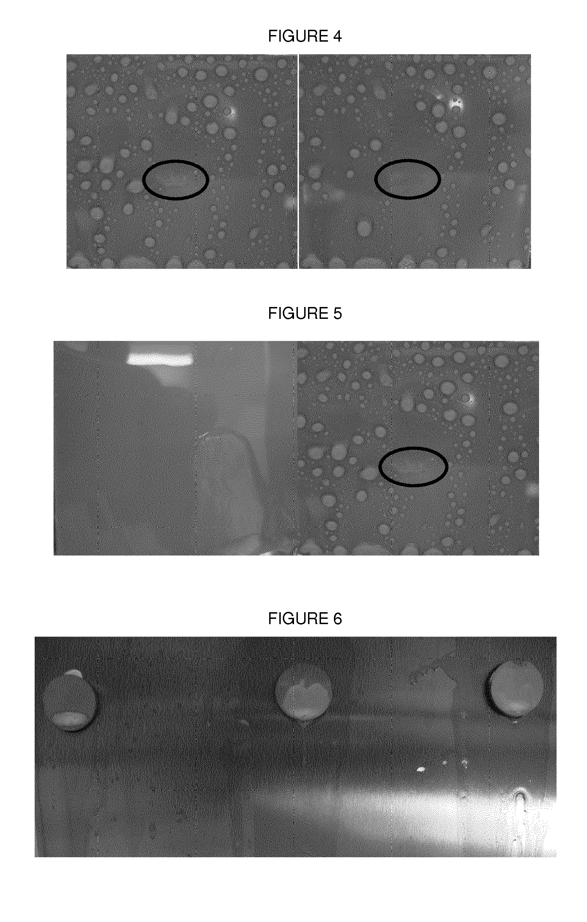

   Figure 4 shows a photograph of a surface coated with epoxy paint with a biofilm of *Pseudomonas aeruginosa,* disposed in vertical position, where an aqueous solution of hydrogen peroxide was applied by spraying, after a period of 30 seconds (left image) and 5 minutes (right image).
Figure 5:
   Figure 5 shows a photograph of the result of the application of the method according to the present invention (left image), or after the application of a simple hydrogen peroxide aqueous solution (right image), for the detection of biofilms of *Pseudomonas aeruginosa* on surfaces coated with epoxy paint, disposed in vertical position, after 5 minutes of reaction.
Figure 6:
   Figure 6 shows a photograph of the result of the application of the method according to the present invention, using a composition without colorant (left image), or with colorant (middle image), or after the application of a simple hydrogen peroxide aqueous solution (right image), for the detection of biofilms of *Pseudomonas aeruginosa* on stainless steel surfaces disposed in vertical position, after 5 minutes of reaction.

### Detailed description of the invention

The object of the present invention is the use of an aqueous composition comprising hydrogen peroxide and a thickening agent for detecting biofilms.

The authors of the present invention have developed a composition based on the combination of hydrogen peroxide and a thickening agent which, surprisingly, is highly effective for the detection of biofilms on any type of surfaces.

The biofilm detecting action of this composition is mainly based on the reaction of biofilms with hydrogen peroxide, by means of the catalase enzyme. Catalases are antioxidant enzymes present in most aerobic and facultative anaerobic microorganisms and as notable exceptions in some anaerobic microorganisms, such as the genus *Bacteroides.* Other enzymes that also react with hydrogen peroxide are peroxidases and hydroxyperoxidases.

These enzymes catalyze the degradation of hydrogen peroxide into oxygen and water, according to the following reaction:

2H₂O₂ **→**2 H₂O + O₂

This decomposition causes effervescence due to the release of oxygen gas, which acts as an indicator of the presence of microorganisms.

Most microorganisms produce one or more catalases that usually respond to the oxidative stress, to hydrogen peroxide or to the presence of other reactive oxygen species, also known as ROS, as described in the article Chelikani et al., Diversity of structures and properties among catalases, CMLS, Cell. Mol. Life Sci., 2004, 61, 192-208.

Generally, most biofilms in the environment are multispecies, i.e. they consist of several species. Among them, it is common to find aerobic bacteria, whose catalase works as an indicator in the use of the invention.

The presence of a thickening substance provides viscosity to the composition, which allows extending the contact time between the product and the biofilm, thus favouring the enzymatic reaction of the catalases, and at the same time increasing the retention power of the oxygen generated when the enzymatic decomposition is performed. All of this allows detecting the presence of biofilms on the surfaces in a highly effective way, with a simple visual inspection.

Advantageously, said composition further contains a surfactant base, which is preferably foaming.

The surfactant base consists of one or more surfactants and performs several functions. On one hand it has a wetting function on the biofilm extracellular matrix, which facilitates the action of the catalases on the hydrogen peroxide. Furthermore, the foaming effect allows a better viewing of the effervescence, as the formed foam contributes to the retention of the bubbles produced by the oxygen released during the reaction.

Also, in a preferred embodiment, the composition further comprises a colorant that facilitates the viewing of the foam formed by the enzymatic reaction.

In the present description as well as in the claims, the singular forms "a" or "an" include also the plural reference unless the context clearly indicates otherwise. Thus, for example, the reference to a "surfactant" also includes two or more surfactants.

### Hydrogen peroxide

Hydrogen peroxide is present in the composition of the invention, and the content thereof is preferably comprised between 0.5% and 50%, more preferably between 4% and 15%, still more preferably between 4.5% and 7%, and still more preferably is approximately 5%, wherein all percentages are expressed by weight based on the total weight of the composition.

The authors of the invention have shown that the composition for detecting biofilm may include a wide content of hydrogen peroxide, preferably comprised between 0.5% and 50%, although the content of hydrogen peroxide can be even higher.

However, it has been observed that with relatively low concentrations of hydrogen peroxide, preferably comprised between 4% and 15%, more preferably between 4.5% and 7%, and still more preferably with a concentration of approximately 5%, the compositions are highly effective for detecting biofilm, without the need to use higher hydrogen peroxide content, which results in a greater safety and a lower cost.

### Thickening agent

The composition for detecting biofilm according to the use of the present invention contains a thickening agent.

Among the thickeners suitable to be incorporated into the compositions according to the use of the present invention are, for example, the thickening agents of natural origin, such as guar gum, xanthan gum, gellan gum, locust bean gum, tragacanth gum, welan gum, pectin, carboxymethylcellulose, alginates, starch, dextrins, carrageenan, bentonite, Konjac gum, among others; as well as homopolymers or copolymers, optionally cross-linked, of olefinically unsaturated carboxylic acids or anhydrides, such as acrylic acid, methacrylic acid or maleic anhydride, among others, or their esters, with free carboxyl groups or in salt form; hydrophobically modified polymer polyols, or urethane polyols; or mixtures thereof. These polymers and copolymers are commercially available, for example, under the trade names Carbopol^{®}, Ultrez^{®}, Acusol^{®}, Acrysol^{®}, Polygel^{®}, Synthalen^{®} and Stabylen^{®}. These polymers can also be used in association with silicates modified with inorganic polyphosphates peptizers, such as the products commercialized under the name Laponite^{®}.

In a preferred embodiment, the thickening agent is selected from the group consisting of xanthan gum, guar gum, gellan gum, locust bean gum, tragacanth gum, carrageenan, Konjac gum, homopolymers or copolymers of olefinically unsaturated carboxylic acids or anhydrides, and cross-linked homopolymers or copolymers of olefinically unsaturated carboxylic acids or anhydrides; more preferably, the thickening agent is selected from xanthan gum, gellan gum, Konjac gum and cross-linked homopolymers or copolymers of olefinically unsaturated carboxylic acids or anhydrides.

A particularly preferred thickening agent is xanthan gum. This product is commercially available through several suppliers, such as, for example, the xanthan gum commercialized by the company CP Kelco, under the trade name Keltrol^{®}.

Another particularly preferred thickening agent are the cross-linked polymers of acrylic acid, for example, those commercialized by the company Lubrizol under the trade name Carbopol^{®}.

Another particularly preferred thickening agent is gellan gum, which is commercially available through several suppliers, such as, for example, the company CP Kelco, under the trade name Kelcogel^{®}.

Another particularly preferred thickening agent is carrageenan, which is commercially available through several suppliers, such as, for example, the company CP Kelco, under the trade name Kelcogel^{®} BF.

Another particularly preferred thickening agent is Konjac gum. This product is also commercially available, for example through the company FMC Corp., under the name Nutricol^{®}.

The incorporation of a thickening agent allows obtaining a composition with a relatively high viscosity. Thus, preferably, the composition of the invention has a viscosity comprised between 100 and 1000 mPa·s, preferably between 300 and 600 mPa·s, and still more preferably of approximately 500 mPa·s.

The content of thickening agent is preferably comprised between 0.05% and 5%, and more preferably comprised between 0.1 % and 2%, where the percentage is expressed by weight based on the total weight of the composition.

### Surfactant

Preferably, the composition for the detection of biofilm further comprises a surfactant.

In said composition, the surfactant exerts on the one hand, a wetting function on the biofilm extracellular matrix, which facilitates the action of the catalases on the hydrogen peroxide. Furthermore, the foaming effect of the surfactant facilitates the viewing of the effervescence, due to the retention of the bubbles produced by the oxygen released during the reaction between hydrogen peroxide and the catalase present in the microorganisms that form the biofilm.

Surfactants, as is well known to those skilled in the art, are amphiphilic substances having a hydrophobic part and a hydrophilic part. This particular chemical structure of surfactants is responsible of their properties, for example, of their wetting action, due to the reduction of the surface tension of the liquid, so that the wetting of the surfaces is favoured. Surfactants also have, in general, foaming properties, favouring the formation of foam, unless they have been designed as low-foam surfactants for applications where foam formation is not desirable.

A variety of surfactants can be obtained commercially from various suppliers such as, for example, the companies Kao Corporation, BASF, Croda, Huntsman, Clariant or Evonik, among others.

The surfactants suitable for the use according to the present invention are preferably foaming surfactants, and are preferably selected from the group consisting of anionic surfactants, amphoteric surfactants, nonionic surfactants, and mixtures thereof.

In a particularly preferred embodiment, the composition according to the use of the present invention comprises a mixture of anionic and nonionic surfactants.

Among the anionic surfactants suitable to be used within the context of the present invention are, for example, soaps, alkylbenzene sulphonic acids and their salts, sulphonated α-olefins, sulphonated paraffins, alkyl sulphates, alkyl ether sulphates, glyceryl ether sulphates, alkyl sulphosuccinates, ethers of carboxylic acids and their salts, alkyl phosphates, alkyl ether phosphates, alkylphenol sulphates, alkylphenol ether sulphates, isethionates, sarcosinates, taurates, and N-acylamino acid salts. To cite only a few examples of this group, among the alkyl ether sulphates is sodium lauryl ether sulphate, as supplied by the company Kao Corporation under the trade name Emal^{®} or the Texapon^{®} product range from the company Cognis (now BASF); the alkyl sulphates are marketed under the trade name Sulfopon^{®} from the company Cognis (now BASF); among the ethers of carboxylic acids are those supplied by the company Kao Corporation under the trade name Akypo^{®}; or among the alkylbenzene sulphonic acids can be mentioned, for example, linear alkylbenzene sulphonic acids (LAS) especially n-dodecylbenzene sulphonic acid and their salts, such as for example that provided by the company Cognis (now BASF) under the trade name Maranil^{®}; and among the α-olefin sulphates, for example, those supplied by the company Clariant under the name Hostapur^{®}.

Preferably the anionic surfactant is an alkyl ether sulphate.

Among the nonionic surfactants suitable to be used within the context of the present invention are, for example, ethoxylated fatty alcohols, ethoxylated fatty acids, ethoxylated alkylphenols, fatty acid alkanolamides, ethoxylated fatty acids alkanolamides , ethoxylated fatty amines, fatty amine oxides, fatty amidoamine oxides, esters of glycerine and fatty acids, sorbitan esters, ethoxylated sorbitan esters, sucrose esters, alkylpolyglycosides, copolymers of ethylene/propylene oxide, among others. For example, among the ethoxylated fatty alcohols can be mentioned those supplied by the company Cognis (now BASF) under the trade name Dehydol^{®}. Among the ethoxylated sorbitan esters can be mentioned those supplied by the company Kao Corporation under the trade name Rheodol^{®}. Among the ethoxylated alkylphenols can be mentioned the products marketed under the trade name Dehydrophen^{®} by the company Cognis (now BASF).

Preferably, the nonionic surfactant is selected from the group consisting of ethoxylated fatty alcohol, ethoxylated sorbitan ester and alkylpolyglycoside; more preferably the nonionic surfactant is an ethoxylated fatty alcohol.

In the book M. Asch and I. Asch, Handbook of Industrial Surfactants, Fourth Edition, Synapse Information Resources, 2005, an extensive list of commercially available surfactants can be found.

The content of surfactant in the composition of the invention is preferably comprised between 3% and 25%, more preferably between 5% and 15%, wherein the percentage is expressed by weight based on the total weight of the composition.

In a preferred embodiment, the composition comprises a mixture of anionic and nonionic surfactants, wherein the content of nonionic surfactant is preferably comprised between 1% and 15%, more preferably between 3% and 10%, and still more preferably between 5% and 8%; and the content of anionic surfactant is preferably comprised between 0.5% and 10% of anionic surfactants, more preferably between 1% and 8%, and still more preferably between 2% and 5%, wherein the percentage is expressed by weight based on the total weight of the composition.

### Colorant

Also, in a preferred embodiment the composition according to the present invention further contains a colorant.

The presence of colorant in the formulation facilitates the viewing of foam formation due to the enzymatic reaction between the catalase and hydrogen peroxide.

The colorant used can be any colorant commonly used in the food or cleaning products, as are well known to the skilled in the art, and which is stable in the composition of the invention.

Preferably the colorant used is very soluble in water, thereby preventing the colouring of the assessed surfaces and reducing the difficulty for its removal, as it happens at present with some of the products available on the market. Additionally, the colorant is capable of colouring the foam formed, to facilitate the viewing of the enzymatic reaction.

Colorant products suitable to be used in the present invention are, for example, anthraquinone dyes, azo dyes or triarylmethane dyes.

Some colorants suitable to be incorporated in the composition of the invention are, for example, Ponceau 4R (also known as Acid Red 18, E124, or Cochineal Red, C.I. 16255, commercially available, for example, under the trade name Sanolin^{®} Ponceau 4RC 82), Reactive Red 180 (C.I. 181055C.I., commercially available, for example, under the name Duasyn^{®} Brilliant Red F3B-SF), Acid Blue 9 (C.I. 42090, commercially available, for example under the trade name Sanolin^{®} Blue AE 90), Acid Yellow 23 (also known as tartrazine, or E102, C.I. 19140, commercially available, for example, under the trade name Sanolin^{®} Tartrazine X90), among others.

When present in the composition, the colorant content is preferably comprised between 0.0001% and 1.0%, more preferably between 0.001% and 0.5%, and still more preferably between 0.005% and 0.2%, expressed by weight based on the total weight of the composition.

### Other components

Additionally, the composition for detecting biofilms according to the use of the present invention can contain other components, such as sequestrants, antioxidants, or pH modifying agents, among others.

In a preferred embodiment, the composition further comprises a sequestering agent.

As it is well known to those skilled in the art, sequestering agents, also known as chelators, are substances capable of forming soluble complexes with metal ions. Specifically, in the detergency field, their presence allows improving the action of surfactants, by removing metal ions from the media, mainly calcium and magnesium, thus diminishing water hardness and reducing the formation of insoluble salts. Furthermore, in the field of compositions comprising hydrogen peroxide, sequestering agents have the function of complexing heavy metal ions that can possibly catalyze the decomposition of hydrogen peroxide in the composition.

Some sequestering agents suitable to be used within the context of the present invention are, for example, polyphosphates, especially tripolyphosphates and alkali pyrophosphates; phosphonates such as HEDP (1-hydroxyethane 1,1-diphosphonic acid), DTPMP (diethylenetriamine pentamethylenphosphonic acid), EDTMP (ethylene diamine tetramethylenphosphonic acid), ATMP (amino trimethylenphosphonic acid) or HDTMP (hexamethylenediamine tetramethylenephosphonic acid), or their alkali salts; hydroxypolycarboxylates, such as citric, tartaric or gluconic acid; aminopolycarboxylates, such as EDTA (ethylenediaminetetraacetic acid), DTPA (diethylenetriamine pentaacetic acid), NTA (nitrile triacetic acid), or GLDA (glutamic acid N,N-diacetic) or their sodium salts; or mixtures thereof.

Preferably the sequestering agent is selected from the group consisting of the phosphonates HEDP, DTPMP, EDTMP, ATMP, HDTMP, or their sodium salts, or mixtures thereof.

When the sequestering agent is present in the composition according to the use of the present invention, the content thereof is comprised between 0.001% and 5%, more preferably between 0.01% and 1.0%, and still more preferably between 0.05% and 0.5%, wherein the percentage is expressed by weight based on the total weight of the composition.

The composition of the invention can also additionally contain stabilizing substances.

Among the stabilizers suitable to be used within the context of the present invention are, for example, 3,5-di-tert-butyl-4-hydroxytoluene (BHT), *tert-butyl* hydroxyanisole (BHA), trimethoxy benzoic acid (TMBA), α-tocopherol, γ-tocopherol, δ-tocopherol, ethoxyquin, 6-hydroxy-2,5,7,8-tetramethylchroman-2-carboxylic acid, ascorbic acid, sorbic acid and its salts, dihydroxyfumaric acid and its salts, lignosulphonic acid and its salts, gallic acid and its alkyl esters, p-anisic acid and its salts, benzoic acid and its salts, 4-hydroxy-TEMPO, p-toluenesulphonic acid and its salts, hydroquinones and their derivatives (such as 2,5-di-*tert-butyl* hydroquinone or DTBHQ, toluhydroquinone or THQ, or mono-*tert-*butyl hydroquinone or MTBHQ) among others, and mixtures thereof.

Preferably BHT is used as a stabilizer.

The amount of stabilizer is preferably comprised between 0.001% and 1%, more preferably comprised between 0.01% and 0.5%, and still more preferably between 0.05% and 0.3%, wherein the percentage is expressed by weight based on the total weight of the composition.

Optionally, the composition for detecting biofilm can contain pH regulating agents, for example, of acid character such as mineral acids or carboxylic acids.

Additionally, the compositions according to the use of the present invention may contain further additional components, such as foam stabilizers or preservatives, for example.

In a particularly preferred embodiment of the present invention, the composition for detecting biofilms comprises:
- between 4% and 15% of hydrogen peroxide, preferably between 4.5% and 7%, and more preferably approximately 5%;
- between 0.05% and 5% of a thickening agent, preferably between 0.1% and 2%;
- between 3% and 25% of surfactant, preferably between 5% and 15%;
- between 0.0001% and 1% of colorant, preferably between 0.001% and 0.5%;
- between 0.001% and 5% of a sequestering agent, preferably between 0.01% and 1.0%; and
- between 0.001% and 1% of stabilizer, preferably between 0.01% and 0.5%, and more preferably between 0.05% and 0.3%.

These percentages are expressed by weight based on the total weight of the composition. The composition is completed with enough quantity of water up to 100%.

In a more preferred embodiment of the present invention, the composition for detecting biofilm comprises:
- between 4% and 15% of hydrogen peroxide, preferably between 4.5% and 10%, more preferably approximately 5%;
- between 0.05% and 5% of a thickening agent, preferably between 0.1% and 2%;
- between 1% and 15% nonionic surfactant, preferably between 3% and 10%;
- between 0.5% and 10% of anionic surfactant, preferably between 1% and 8%;
- between 0.0001% and 1% of colorant, preferably between 0.001% and 0.5%;
- between 0.001% and 5% of a sequestering agent, preferably between 0.01% and 1.0%; and
- between 0.001% and 1% of stabilizer, preferably between 0.01% and 0.5%, and more preferably between 0.05% and 0.3%.

These percentages are expressed by weight based on the total weight of the composition. The composition is completed with enough quantity of water up to 100%.

In the context of the description, the weight percentages relate to the weight of active material of each component.

In a particularly preferred embodiment the thickening agent is selected from the group consisting of xanthan gum, gellan gum, konjac gum and cross-linked polymers of acrylic acid; the anionic surfactant is an alkyl ether sulphate; and the nonionic surfactant is an ethoxylated fatty alcohol.

The preparation of the composition according to the use of the present invention can be performed by methods well known in the detergents and cleaning products technology field. For example, it may be prepared by dissolving the different components in water, under stirring until obtaining a homogeneous solution.

### Method for detecting biofilm

Another aspect of the object of the present invention is a method for detecting biofilms on a surface, comprising the following steps:
(i) spraying onto the surface a composition comprising hydrogen peroxide and a thickening agent;
(ii) allowing the composition to act for a period of time comprised between 30 seconds and 20 minutes; and
(iii) visually inspecting the appearance of bubbling.

The spraying action required in step (i) refers to the action of applying a thin layer of the composition on the surface, spread on droplets, to which end any standard spray device can be used, for example, a gun-shaped manual spray, or by using an impeller pump.

The composition applied in step (i) preferably has all the features as described above.

The appearance of bubbles is indicative of the enzymatic reaction of the catalases present in the biofilm and therefore allows detecting its presence on the surface by means of a simple visual inspection.

The simplicity of this method has the advantage of allowing its application *in situ* for detecting biofilms on any surface, both in industrial facilities as well as in the domestic environment, and allows fast and efficient detection of biofilms.

This method allows the detection of biofilms, both monospecies and multispecies, which are catalase-positive. Examples of microorganisms that have some type of catalase are, for example, within the bacterial genus, *Salmonella, Listeria, Escherichia, Staphylococcus, Micrococcus, Helicobacter, Enterococcus, Proteus, Pseudomonas,* among others; and within the fungal genus, *Saccharomyces, Candida,* Aspergillus, *Fusarium, Botrytis, Cladosporium,* among others, as described in the article Beltrán-García et al., Catalasas de hongos fitopatogenos: Factores de virulencia y resistencia a los fungicidas?, Rev. Mex. Fitopatol., 2006, 24(1), 50-58.

In the practice, this method is suitable for the detection of virtually all the biofilms usually present in the industry and house environments, since generally they always include one bacterium that is catalase-positive. Since most environmental biofilms are of the multispecies type, it is sufficient that the biofilm comprises at least one catalase-positive species to be sensitive to the detection method.

As for the surface, virtually any type of surface is suitable for the biofilm detection method of the present invention to be applied, either metallic, such as aluminium, chromium, steel, or stainless steel; or surfaces such as glass, ceramic or porcelain; as well as of any type of plastic, such as thermoplastic or thermosetting polymers, for example, epoxy resins; among other possible materials.

The surface to be analyzed can be in any disposition, either horizontal, vertical, or in ceilings.

Figure 1 shows a photograph of a stainless steel surface, disposed in horizontal position, on which the method of the invention was applied for the detection of *Pseudomonas aeruginosa* biofilm.

Similarly, in figure 2 a photograph is shown of the application of the method of the invention for detecting the same kind of biofilm, also on a stainless steel surface, but this time disposed in vertical position.

As can be seen in those photographs, after spraying the product of the invention on the surface, the presence of biofilm causes the appearance of bubbling, which is easily observable by simple visual inspection.

The period of time required in the step (ii) of the method, during which the composition is allowed to act, ranges from 30 seconds to 20 minutes, and it will depend on the enzymatic production of catalase and, therefore, on the density of the biofilm on the surface. Preferably, the operation time is between 30 seconds and 5 minutes, given that a longer period of time is not necessary for effective detection of the biofilm, but it can optionally be allowed to act for a longer period, up to about 20 minutes, without prejudice to efficiency. The method developed in the present invention has the advantage that it is valid for the detection of biofilms on any surface and in any environment. It can be especially useful in those areas where hygiene requirements are particularly strict, as the food industry or the health environment.

In particular, the present method for the detection of biofilm may be particularly advantageous as an assistant for the establishment of the Critical Control Points (CCP) in the surfaces of the agri-food industry.

### Example 1: Composition for detecting biofilm

7 formulations (A to G) of compositions for the detection of biofilm were prepared, according to the use of the present invention, using the proportions detailed in the following table:

| | Compositions (% of active material by weight) | | | | | | |
|---|---|---|---|---|---|---|---|
| | **A** | **B** | **C** | **D** | **E** | **F** | **G** |
| Hydrogen peroxide | 0.5 | 1 | 3 | 5 | 7 | 10 | 15 |
| Thickener | 0.10 | 0.15 | 0.20 | 0.25 | 0.75 | 1.00 | 1.50 |
| Ethoxylated fatty alcohol | 1 | 5 | 5 | 7 | 8 | 10 | 14 |
| Alkyl ether sulphate | 14 | 10 | 8 | 3 | 5 | 5 | 1 |
| Phosphonates | 0 | 0.025 | 0.05 | 0.2 | 25 | 3 | 5 |
| Colorant | 0 | 0.0001 | 0.001 | 0.01 | 0.02 | 0.05 | 0.1 |
| BHT | 0.1 | 0.1 | 0.1 | 0.1 | 0.1 | 0.1 | 0.1 |
| Water | q.s. 100 | | | | | | |

All the compositions obtained had viscosity values comprised between 300 and 600 mPa·s.

### Example 2: Efficacy assays of the biofilm detection

The effectiveness of the compositions of the invention was determined for biofilms prepared following procedures well known to the skilled in the art such as those described in, for example, the articles Fuster et al., Effect of different environmental conditions on the bacteria survival on stainless steel surfaces, Food Control, 2008, 19 (3), 308-314 and Montanez-Izquierdo et al., Use of epifluorescence microscopy to assess the effectiveness of phage P100 in controlling Listeria monocytogenes biofilms on stainless steel surfaces, Food Control, 2012, 23 (2), 470-477. To this end, tests with different conditions of humidity, temperature and time were performed, selecting those that allowed optimum adhesion of the microorganism to the different test surfaces and that, furthermore, allowed to reproduce the biofilm formation in a homogeneous and repetitive way.

Biofilms were designed with microorganisms of general concern in the food and hospital environments, such as *Listeria monocytogenes, Salmonella* spp, *Escherichia coli, Cronobacter sakazakii, Pseudomonas* spp, *Staphylococcus aureus,* and *Candida albicans.* Moreover, as a control of negative catalase, *Lactobacillus* spp.

Next, the 7 compositions prepared in Example 1 (compositions A to G), all of them within the scope of the present invention, were tested. To this end, the compositions were sprayed over the biofilms on the surfaces mentioned, both in horizontal and vertical disposition. After spraying, a visual inspection was performed at different times, and thus the production of an easily visible effervescence was observed, indicating the presence of biofilms.

The formulas comprising colorant (B to G) were more effective in terms of facilitating the viewing of the generated effervescence.

Figures 1 and 2 show two photographs with the result of the assay performed with the formulation B, on the surface disposed horizontally and vertically, respectively. The effervescence, indicative of the presence of biofilm, can be observed with the naked eye.

### Example 3: Comparative efficacy tests

Surfaces coated with epoxy paint were used for the development of *Pseudomonas aeruginosa* biofilms.

Figure 3 shows two surfaces coated with epoxy paint on whose surface *Pseudomonas aeruginosa* biofilms have been developed. The surfaces are disposed horizontally, and in figure 3A they are shown 30 seconds after having sprayed the composition according to the present invention (left image) and a simple solution composed of water and hydrogen peroxide with the same concentration (right image).

Figure 3B shows a photograph with the same surfaces of figure 3, but after 5 minutes of reaction.

In Figure 3C a photograph is shown with the same surfaces of figure 3, but after 15 minutes of reaction.

The bubbling generated with the composition according to the present invention becomes more visible, because it is stronger, more compact, and remains over the time with respect to that of the simple solution, which it is shown weak, disperse and disappearing over the time.

In figure 4 a photograph is shown of a surface coated with epoxy paint with a *Pseudomonas aeruginosa* biofilm, disposed vertically, where the simple solution composed of water and hydrogen peroxide used in the test of figure 3 was applied by spraying. The reaction times are of 30 seconds and 5 minutes for the left and right images, respectively.

A weak initial bubbling can be seen after 30 seconds (pointed out by the oval in the left image) which remains hardly visible as time goes on (pointed out by the oval in the right image).

In figure 5 a photograph is shown of two surfaces coated with epoxy paint with *Pseudomonas aeruginosa* biofilms, disposed in vertical position, after 5 minutes of reaction after spraying the composition according to the present invention (left image) and a simple solution composed of water and hydrogen peroxide with the same concentration (right image).

In the surface coated with epoxy paint disposed in vertical position, a big difference can be seen in the bubbling production and its permanence over the time with the composition according to the invention in comparison with the simple solution, where the bubbling production is seen only weakly, since it slides and quickly disappears.

In figure 6 a photograph is shown of three stainless steel surfaces with *Pseudomonas aeruginosa* biofilms, disposed in vertical position, after 5 minutes of reaction after spraying the simple solution composed of water and hydrogen peroxide (right surface), a composition according to the present invention (central surface) and a composition according to the present invention without colorant (left surface).

Here it can be appreciated that, from a reasonable distance, of less than one meter, the biofilm of the central image can be better viewed due to the bubbling production and the colour, even though the coloration is not intense, but nevertheless it helps to its easy detection with respect to the biofilm on the right, where there is a thickening agent but no colorant.

## Claims

1. Use of an aqueous composition comprising hydrogen peroxide and a thickening agent for detecting biofilms.

2. Use according to claim 1, **characterized in that** the content of hydrogen peroxide is comprised between 0.5% and 50%, expressed by weight based on the total weight of the composition.

3. Use according to claim 2, **characterized in that** the content of hydrogen peroxide is comprised between 4.5% and 7%, expressed by weight based on the total weight of the composition.

4. Use according to any of claims 1 to 3, **characterized in that** the thickening agent is selected from the group consisting of xanthan gum, guar gum, gellan gum, locust bean gum, tragacanth gum, carrageenan, Konjac gum, homopolymers or copolymers of olefinically unsaturated carboxylic acids or anhydrides, and cross-linked homopolymers or copolymers of olefinically unsaturated carboxylic acids or anhydrides.

5. Use according to claim 4, **characterized in that** the thickening agent is selected from xanthan gum, gellan gum, Konjac gum and cross-linked homopolymers or copolymers of olefinically unsaturated carboxylic acids or anhydrides.

6. Use according to any of claims 1 to 5, **characterized in that** the content of thickening agent is comprised between 0.05% and 5%, expressed by weight based on the total weight of the composition.

7. Use according to any of claims 1 to 6, **characterized in that** the composition further contains a surfactant.

8. Use according to claim 7, **characterized in that** the surfactant is selected from the group consisting of anionic surfactants, amphoteric surfactants, nonionic surfactants, and mixtures thereof.

9. Use according to claim 8, **characterized in that** the composition comprises a mixture of anionic and nonionic surfactants.

10. Use according to claim 9, **characterized in that** the anionic surfactant is an alkyl ether sulphate and the nonionic surfactant is selected from the group consisting of ethoxylated fatty alcohol, ethoxylated sorbitan ester and alkylpolyglycoside.

11. Use according to any of claims 7 to 10, **characterized in that** content of surfactant is comprised between 3% and 25%, expressed by weight based on the total weight of the composition.

12. Use according to claims 9 or 10, **characterized in that** the content of nonionic surfactant is comprised between 1% and 15%, and the content of anionic surfactant is comprised between 0.5% and 10%, expressed by weight based on the total weight of the composition.

13. Use according to any of claims 1 to 12, **characterized in that** the composition further contains a colorant.

14. Use according to any of claims 1 to 13 **characterized in that** the composition further contains a sequestering agent.

15. Use according to claim 1, **characterized in that** the composition comprises:
- between 4% and 15% of hydrogen peroxide;
- between 0.05% and 5% of a thickening agent;
- between 3% and 25% of surfactant;
- between 0.0001% and 1% of colorant;
- between 0.001% and 5% of a sequestering agent; and
- between 0.001 % and 1 % of stabilizer,
wherein the percentages are expressed by weight based on the total weight of the composition.

16. Use according to claim 1, **characterized in that** the composition comprises:
- between 4% and 15% of hydrogen peroxide;
- between 0.05% and 5% of a thickening agent;
- between 1% and 15% of nonionic surfactant;
- between 0.5% and 10% of anionic surfactant;
- between 0.0001% and 1% of colorant;
- between 0.001% and 5% of a sequestering agent; and
- between 0.001 % and 1 % of stabilizer,
wherein the percentages are expressed by weight based on the total weight of the composition.

17. Method for detecting biofilms on a surface, comprising the following steps:
(i) spraying onto the surface a composition comprising hydrogen peroxide and a thickening agent;
(ii) allowing the composition to act for a period of time comprised between 30 seconds and 20 minutes; and
(iii) visually inspecting the appearance of bubbling.

18. Method according to claim 17, **characterized in that** the composition sprayed in step (i) is as defined in any of claims 2 to 16.
